# EUROPEAN PATENT SPECIFICATION

(11) **EP 2 268 315 B1**
(45) Date of publication and mention of the grant of the patent: **31.07.2019**
(21) Application number: 09727280.1
(22) Date of filing: 31.03.2009
(51) Int. Cl.: A61K 47/60

(54) **DRUG CONJUGATES WITH POLYGLYCEROLS**
WIRKSTOFFKONJUGATE MIT POLYGLYCEROLEN
CONJUGUÉS DE MÉDICAMENTS AVEC POLYGLYCÉROLS

(30) Priority: 31.03.2008 EP 08006471; 06.02.2009 EP 09001693
(43) Date of publication of application: 05.01.2011
(73) Proprietor: Freie Universität Berlin, 14195 Berlin (DE); Vergell Medical S.A., 1204 Geneva (CH)
(72) Inventor: KRATZ, Felix, 79106 Freiburg (DE); HAAG, Rainer, 14195 Berlin (DE); CALDERON, Marcelo, 14195 Berlin (DE)
(74) Representative: Müller-Boré & Partner Patentanwälte PartG mbB
(86) International application number: PCT/EP2009/002346
(87) International publication number: WO 2009/121564

(56) References cited:
- WO-A-2005/028539
- WO-A-2006/018295
- WO-A-2006/136450
- PARAG KOLHE ET AL: "Hyperbranched Polymer-Drug Conjugates with High Drug Payload for Enhanced Cellular Delivery" PHARMACEUTICAL RESEARCH, KLUWER ACADEMIC PUBLISHERS-PLENUM PUBLISHERS, NE, vol. 21, no. 12, 1 December 2004 (2004-12-01), pages 2185-2195, XP019370658 ISSN: 1573-904X
- HAAG R ET AL: "POLYMER THERAPEUTICS: CONCEPTS AND APPLICATIONS" ANGEWANDTE CHEMIE. INTERNATIONAL EDITION, VCH VERLAG, WEINHEIM, DE, vol. 45, no. 8, 13 February 2006 (2006-02-13), pages 1198-1215, XP009066269 ISSN: 0570-0833
- LEE CAMERON C ET AL: "A single dose of doxorubicin-functionalized bow-tie dendrimer cures mice bearing C-26 colon carcinomas" PROCEEDINGS OF THE NATIONAL ACADEMY OF SCIENCES OF THE UNITED STATES OF AMERICA, vol. 103, no. 45, November 2006 (2006-11), pages 16649-16654, XP002528429 ISSN: 0027-8424

## Description

The present invention relates to a drug polymer conjugate comprising a pharmaceutically and/or diagnostically active compound bound to a dendritic polyglycerol core having a polyethylene glycol shell.

Most of the drugs used at present are compounds having low molecular weights and exhibit, when systemically administered to a patient, a high plasma clearance or total body clearance. Furthermore, said low molecular weight compounds show a high tendency to penetrate body tissues by diffusion, resulting in a uniform biodistribution. These are the two main reasons why only small quantities of the drug reach the site of action and, due to distribution over healthy tissues of the body, said drugs give rise to problematic side-effects. These disadvantages are of particular concern for those drugs having a high cytotoxic potential, such as cytotoxic agents, immunosuppressive agents or virostatic agents.

Several strategies have been pursued for improving the selectivity of low molecular weight drugs and thus to increase the concentration of the active agent in the desired tissue, while the concentration of the same is decreased in healthy tissues for a reduction of side-effects. These include active and passive targeting approaches with antibodies, serum proteins, synthetic polymers, liposomes and nanocarriers (R. Haag, F. Kratz (2006): Polymer Therapeutics: Concepts and Applications, Angewandte Chemie, Int. Ed., 45, 1198-1215). In the design of drug conjugates with synthetic polymers there is a great need of using polymers with a low polydispersity and high uniformity, high water-solubility and well defined functional groups for drug conjugation.

Conjugates comprising a polyglycerol core and pharmaceutically and/or diagnostically active agents bound to said core using a polyethylene glycol comprising linker are disclosed in WO2005/028539 and in WO2006/136450.

In view of the above, the technical problem underlying the present invention is to provide novel prodrugs which should comprise a suitable carrier having at least one, and preferably two or more binding site(s) and one or more pharmaceutically and/or diagnostically active compounds bound thereto, whereby improved therapeutic properties should be obtained.

According to the present invention, the above-described technical problem is solved by providing a drug polymer conjugate, comprising a dendritic polyglycerol core with a polyethylene glycol shell, and further comprising at least one pharmaceutically and/or diagnostically active compound, wherein the pharmaceutically and/or diagnostically active compound is bound to said dendritic polyglycerol core through a polymer-binding spacer comprising a polymer-binding moiety and a cleavable linker, and wherein the pharmaceutically and/or diagnostically active compound is not bound to the polyethylene glycol shell.

The expression "drug polymer conjugate" of the present invention is not specifically restricted and includes any compound wherein a pharmaceutically and/or diagnostically active compound is bound to a dendritic polyglycerol. In this context, the term "dendritic polyglycerol" as used herein includes any substance which contains at least two glycerol units in its molecule and wherein said molecule is characterized by a branched structure. According to the present invention, the term "glycerol unit" does not only relate to glycerol itself but also includes any subunits which are based on glycerol, such as for example

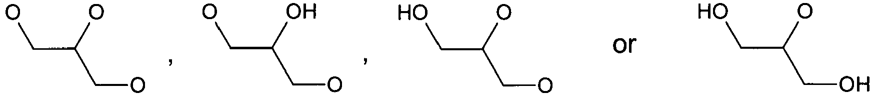

Preferably, the dendritic polyglycerol includes three or more, more preferably four or more and most preferably five or more of said glycerol units, either directly bound to each other, in form of different parts of a molecule or as a combination of both. The dendritic polyglycerol structure can be obtained by either a perfect dendrimer synthesis, a hyperbranched polymer synthesis or a combination of both (R. Haag, A. Sunder, J.-F. Stumbé, (2000): An approach to glycerol dendrimers and pseudo-dendritic polyglycerols, J. Am. Chem. Soc. 122, 2954-2955). The dendritic polyglycerol can also be further functionalized with various reactive goups, such as amines, halides, hydrazines, sulfonates, thiols etc. (S. Roller, H. Zhou, R. Haag (2005): High-loading polyglycerol supported reagents for Mitsunobu and acylation reactions and other useful polyglycerol derivatives Molecular Diversity, 9, 305-316).

The expression "core" as used herein is not specifically restricted and relates to the central or inner part of a molecular structure having for example a spherical or cylindrical.

The expression "polyethylene glycol" means, according to the present invention, any molecule which contains at least two ethylene glycol units, i.e. 1,2-ethanediol units, and is not restricted in any other way. For example, the term polyethylene glycol includes oligoethylene glycols having 2 to 12 ethylene glycol units, or polyethylene glycols having up to several hundred ethylene glycol units. Moreover, according to the present invention, the expression polyethylene glycol also includes mixtures of polyethylene glycol groups comprising different numbers of ethylene glycol units. Additionally, the polyethylene glycols of the present invention are not limited to polyethylene glycol as such but may contain one or more other chemical groups which may, for example, add functionality to the polyethylene glycol such as binding or cleavage sites. Further examples of such chemical groups are functional end groups or groups which originate from the synthesis of said polyethylene glycols or from purification and isolation processes.

According to the present invention, said polyethylene glycol comprises from 2 to 1000, preferably from 3 to 500 and more preferably from 4 to 100 ethylene glycol units.

The term "shell" as used herein relates to a portion of a molecule or a conjugated set of molecules which is in a more peripheral position in respect to a core or central portion of the same molecule. The polyethylene glycol shell of the present application may consist of only one type of polyethylene glycol molecules, but may also consist of a variety of different polyethylene glycols. For example, the polyethylene glycol shell of the present invention may contain a distribution over different polyethylene glycol molecules, as e.g. present in commercially available polyethylene glycols, wherein the mean molecular weight is given.

The term "prodrug" as used herein relates to certain forms of the drug polymer conjugate of the present invention and means any form thereof which can be administered to an organism, such as a human, in an inactive or less active form and is converted, e.g. by metabolization, into the active form. Said conversion of the prodrug into the active form is not specifically restricted and includes any chemical and/or physical alteration of the prodrug which occurs after administration, such as for example release of an active part of the prodrug at the location of action.

The expression "pharmaceutically and/or diagnostically active compound" means any compound which brings about a pharmacological effect either by itself or after its conversion in the organism in question, and thus also includes the derivatives from these conversions. The pharmacological effect of the pharmaceutically and/or diagnostically active compound according to the present invention can be a single effect only, e.g. a cytostatic effect, or a broad pharmacological spectrum of action, such as a cytostatic and antiphlogistic effect at the same time.

The expression "cleavable linker" means any linker which can be cleaved physically or chemically. Examples for physical cleavage may be cleavage by light, radioactive emission or heat, while examples for chemical cleavage include cleavage by redox-reactions, hydrolysis, pH-dependent cleavage or cleavage by enzymes.

Examples of said linkers include cleavable linkers comprising one or more hydrolytically cleavable bonds, the hydrolysis of which releases the pharmaceutically and/or diagnostically active compounds. Examples for hydrolytically cleavable bonds are ester bonds or metal-complex bonds, such as are present in platinum-dicarboxylate complexes, where a diaminediaquoplatinum(II) complex is liberated. Furthermore, the cleavable linker may be cleavable by an enzyme. For example, the cleavable linker of the present invention may contain at least one peptide bond which preferably lies within a cleavable peptide sequence of a protease. At least one peptide bond can therefore be implemented by the insertion of a respective peptide sequence into the cleavable linker. The cleavabe linker may also contain one or more self-immolative linkers that produces after peptide cleavage a labile self-immolative spacer drug derivative that in turn hydrolyses in a spontaneous reaction and releases the pharmaceutically and/or diagnostically active compound. One example of a self-immolative linker is a p-aminobenzyloxycarbonyl (PABC) spacer or any of the following wherein R is a functional group selected from H, Cl, Br, I, F, NO₂, CN, OH or an aliphatic or aromatic moiety and the trigger is a peptide that acts as a protease substrate. Specific examples of such a linker are listed in the following Table 1:

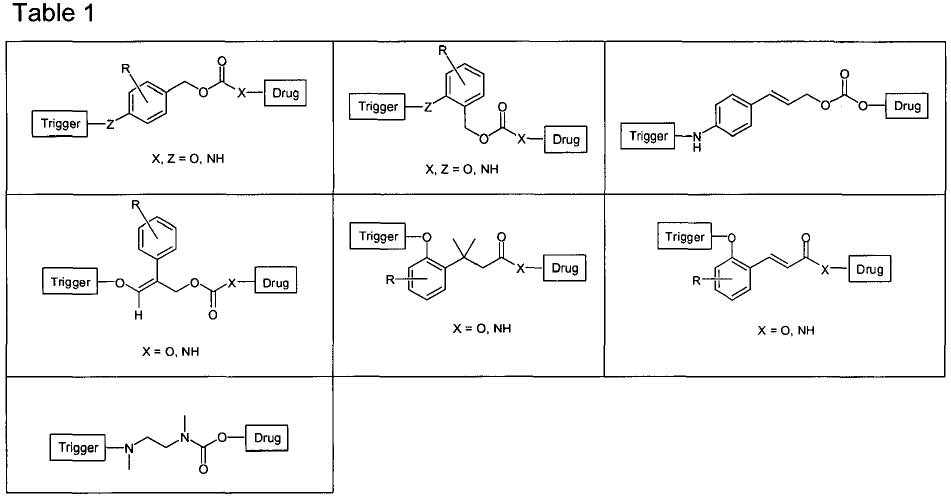

Suitable enzymes are, for example, proteases and peptidases, for example matrix metalloproteases (MMP), cysteine proteases, serine proteases and plasmin activators, which are formed or activated in intensified manner in diseases such as rheumatoid arthritis or cancer, leading to excessive tissue degradation, inflammations and metastasis. Preferred examples of proteases according to the present invention are in particular MMP2, MMP3 and MMP9. Furthermore, the cleavable linker according to the present invention preferably contains at least one acid-labile bond. Examples of acid-labile bonds are ester, acetal, ketal, imine, hydrazone, carboxylhydrazone and sulfonylhydrazone bonds and bonds containing a trityl group.

The expression "polymer-binding spacer" as used herein is not specifically restricted as long as it comprises a polymer binding moiety and a cleavable linker. The polymer-binding spacer may therefore be any molecule which is on the one hand capable of binding to a polymer and on the other hand capable of binding to said pharmaceutically and/or diagnostically active compound, and which can be cleaved in order to separate or release the active compound from the polymeric carrier.

According to another embodiment of the present invention, there is provided a drug polymer conjugate as defined above, wherein the at least one pharmaceutically and/or diagnostically active compound is selected from the group consisting of a cytostatic agent, a cytokine, an immunosuppressant, an antirheumatic, an antiphlogistic, an antibiotic, an analgesic, a virostatic, and an antimycotic agent, a transcription factor inhibitor, a cell cycle modulator, an MDR modulator, a proteasome or protease inhibitor, an apoptosis modulator, an enzyme inhibitor, an angiogenesis inhibitor, a hormone or hormone derivative, a radioactive substance, a light emitting substance, and a light absorbing substance.

A specific embodiment of the present invention relates to the above-defined drug polymer conjugate, wherein the at least one pharmaceutically and/or diagnostically active compound is a cytostatic agent selected from the group consisting of N-nitrosoureas; the anthracyclines doxorubicin, daunorubicin, epirubicin, idarubicin, mitoxantrone and ametantrone, 2-pyrollinoanthracyclines, morpholinoanthracyclines, diacetatoxyalkylanthracyclines, and any derivatives thereof; the alkylating agents chlorambucil, bendamustine, melphalan, and oxazaphosphorines, and any derivatives thereof; the antimetabolites 5-fluorouracil, 2'-deoxy-5-fluorouridine, cytarabine, cladribine, fludarabine, pentostatine, gemcitabine and thioguanine, and any derivatives thereof; the folic acid antagonists methotrexate, raltitrexed, pemetrexed and plevitrexed, the taxanes paclitaxel and docetaxel, and any derivatives thereof; the camptothecins topotecan, irinotecan, 9-aminocamptothecin and camptothecin, and any derivatives thereof; the *Vinca* alkaloids vinblastine, vincristine, vindesine and vinorelbine, and any derivatives thereof; calicheamicins and any derivatives thereof; maytansinoids and any derivatives thereof; auristatins and any derivatives thereof; bleomycin, dactinomycin, plicamycin, mitomycin C and cis-configured platinum(II) complexes.

In a further embodiment of the above-defined drug polymer conjugate, the pharmaceutically and/or diagnostically active compound comprises one or more radionuclide(s), one or more positron emitter(s), one or more NMR contrast agent(s), one or more fluorescent compound(s), or one or more near infrared contrast agent(s).

According to another embodiment of the present invention, in the drug polymer conjugate as defined above, said cleavable linker can be cleaved hydrolytically and/or enzymatically and/or pH-dependently.

In one embodiment, the polymer-binding moiety of the above-defined drug polymer conjugate is selected from the group consisting of a maleinimide group, a halogenacetamide group, a halogenacetate group, a pyridylthio group, a vinylcarbonyl group, an aziridin group, a disulfide group, a substituted or unsubstituted acetylene group, and a hydroxysuccinimide ester group.

According to another aspect of the present invention, there is provided a pharmaceutical composition comprising the above-defined drug polymer conjugate, and optionally a pharmaceutically acceptable carrier and/or a pharmaceutically acceptable adjuvant and/or diluent.

The pharmaceutical composition of the present invention may for example contain solvents and diluents such as physiological saline or a solution containing any pharmaceutically acceptable buffer. Moreover, the pharmaceutical composition of the present invention may be in any form suitable for administration to a patient, for example in an injectable form, as a tablette or a capsule, or as a composition for inhalation.

Moreover, the above-defined pharmaceutical composition can be for the treatment or prevention of a disorder selected from the group consisting of e.g. cancer, autoimmune diseases, acute or chronic inflammatory diseases or diseases caused by viruses and/or microorganisms.

In a specific embodiment of the present invention, the pharmaceutical composition as defined above is for the treatment or prevention of a disorder selected from the group consisting of cancer, autoimmune diseases, acute or chronic inflammatory diseases and diseases caused by viruses and/or microorganisms.

A further aspect of the present invention relates to a kit comprising the above-defined drug polymer conjugate, or the pharmaceutical composition as defined above, and optionally one or more pharmaceutically acceptable adjuvants and/or diluents.

Another aspect of the present invention relates to the drug polymer conjugate as defined above for use in treating a patient suffering from a disorder selected from the group consisting of cancer, autoimmune diseases, acute or chronic inflammatory diseases or diseases caused by viruses and/or microorganisms.

Moreover, according to the present invention, one or more of the different above-mentioned pharmaceutically and/or diagnostically active compounds may be bound to the same carrier molecule. Additionally, each of the above-mentioned one or more pharmaceutically and/or diagnostically active compounds may be bound multiple times to the same carrier.

The drug polymer conjugate according to the present invention advantageously comprises one or more pharmaceutically and/or diagnostically active compounds of the same or different kind, thus enabling the combination of a variety of effective compounds at the same time, while side-effects, such as toxicity, are effectively reduced by a polyethylene glycol shell-like structure. Preferably, depending on the number of binding sites within the carrier molecule, the drug polymer conjugate may contain two or more, preferably three or more and more preferably four or more molecules of said pharmaceutically and/or diagnostically active compounds. On the one hand, such a system can advantageously be exploited to yield highly loaded prodrugs comprising in only one of said conjugate molecules a multiplicity of e.g. two, three, four or even more of the same pharmaceutically and/or diagnostically active compound for achieving an enhanced local concentration and thus a surprisingly improved efficacy. On the other hand, prodrugs can be designed which comprise an advantageous combination of different pharmaceutically and/or diagnostically active compounds. By using such drug polymer conjugates comprising combinations of e.g. two, three, four or more different active compounds, highly individualized modes of treatment and/or prevention are provided which allow a simultaneous effect of each of the active compounds to take place.

The above-described highly advantageous effects, namely a high loading, an increased biocompatibility (i.e. low toxicity) due to the polyethylene glycol shell and any desired combination of active compounds can be combined in the drug polymer conjugates of the present invention, thereby providing a surprisingly efficient and versatile tool in treating and/or preventing disorders in a patient such as a human or an animal.

According to the present invention, the unique structure of the drug polymer conjugate enables a surprisingly effective treatment of disorders in humans and animals while a variety of side effects can be advantageously reduced to a minimum. The dendritic core of the conjugate according to the present invention allows the binding of a polyethylene glycol shell and of one or more pharmaceutically and/or diagnostically active substances to yield a new class of highly versatile prodrugs. The polyethylene glycole shell ensures a favorable biodistribution and the predetermined breaking point is chemically well-defined because the prodrug is coupled to the dendritic polymer in a final step during synthesis.

The Figure shows:

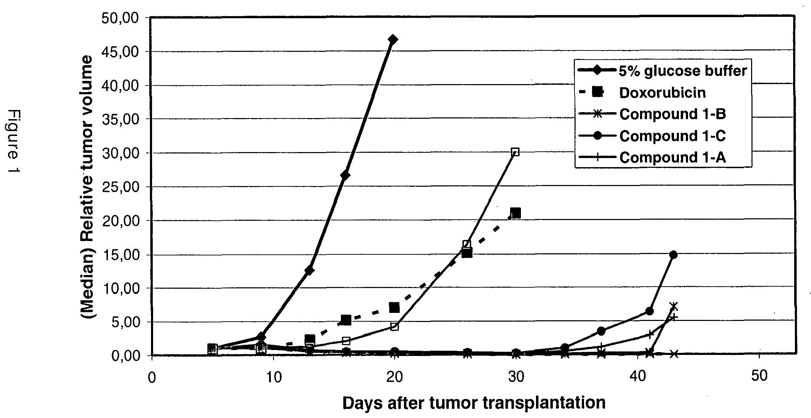

Fig. 1 shows Curves depicting tumor growth inhibition of subcutaneously A2780 xenografts under therapy with doxorubicin and compounds 1-A, 1-B, and 1-C; * significant to control; p < 0.05.

The present invention will be further illustrated in the following examples.

### EXAMPLES

Example 1: Preparation of 4 conjugates with polyglycerolamine (PG 10 kDa and 20% of amine loading) and the (6-maleimidocaproyl)hydrazone derivative of doxorubicin (DOXO-EMCH) using iminothiolane as the thiolating agent and maleimido-polyethylene glycol (PEG 2kDa and 5kDa) as decorating agent - compounds 1-A, 1-B, 1-C and 1-D.

The conjugation reaction was performed at room temperature with vigorous stirring for 90 minutes. To four different flasks, containing 21 mL of a solution of polyglycerolamine (10 mg/mL) in sterile 5 mM EDTA, 50 mM sodium phosphate pH 7.0 were added 43 mL of a solution of 2-iminothiolane (2 mg/mL in the same solvent system). After twenty minutes, a solution of DOXO-EMCH (10 mg/mL in 10 mM sodium phosphate buffer, pH 5.8) was added (a. 8.2 mL; b. 16.4 mL; c. 8.2 mL; d. 1.6 mL). After ten minutes, solutions with a concentration of 200 mg/mL of PEG were added (a. 6.3 mL PEG 2 kDa; b, c and d. 15 mL PEG 5 kDa), and the resulting solutions were stirred for one hour. The solutions were concentrated with CENTRIPREP-10-concentrators from Amicon, FRG (20 min at 4° C and 4000 rpm) to a volume of approximately 5 mL. The PG-DOXO conjugates were purified by gel-filtration through a Sephadex G-25 column (Amersham) with 10 mM sodium phosphate buffer, pH 7.0 yielding 20-50 mL of a red solution. A second concentration with CENTRIPREP was made and finally the conjugates were lyophilized at -60°C for 16 hours to yield a red powder. Yields obtained were between 54% and 74%.

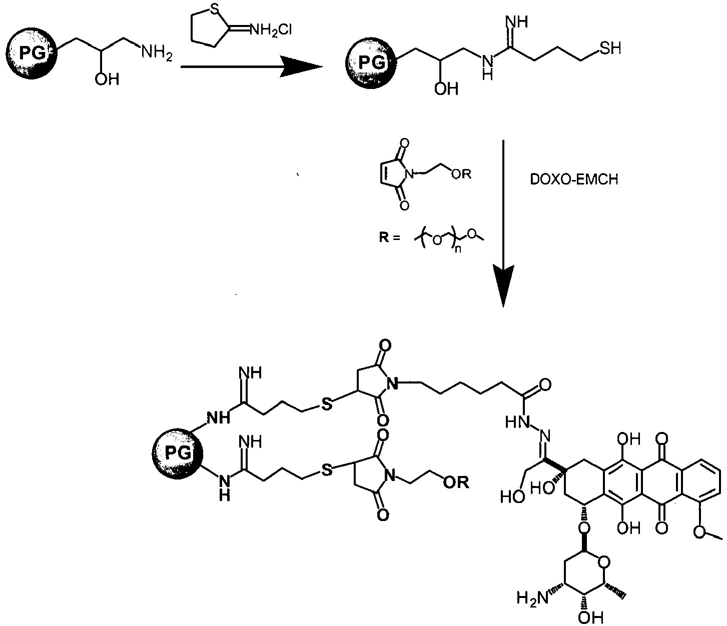

Table 1 summarizes data for the compounds 1-A, 1-B, 1-C and 1-D that were obtained.

**Table 1: Molecular weight, doxorubicin content, yields and structure of compounds 1-A, 1-B, 1-C and 1-D**

| **Sample** | **Theoretical MW** | **Doxorubicin contain in weight %** | **amount recovered/yield** | **Conjugate - idealized structure** |
|---|---|---|---|---|
| Compound 1-A | ∼ 52 kDa | 3.88 | 718 mg / 54% | 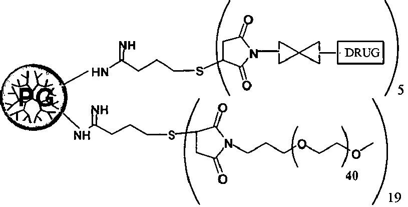 |
| Compound 1-B | ∼ 91 kDa | 2.54 | 2.035 g / 74% | 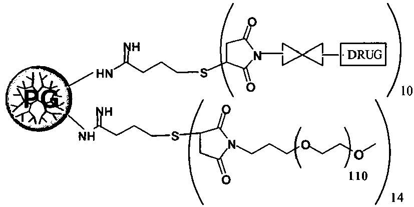 |
| Compound 1-C | ∼ 112 kDa | 1.67 | 1.622 g / 60.75 % | 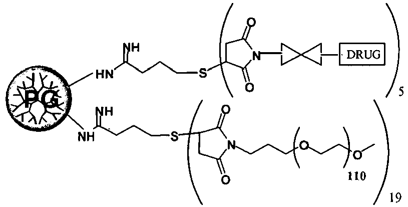 |
| Compound 1-D | ∼ 130 kDa | 0.45 | 1.6678 g / 64% | 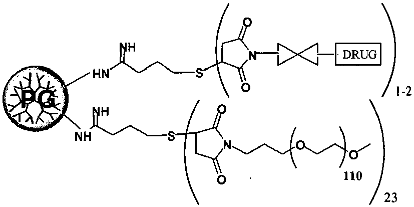 |

Example 2: pH-dependant stability studies with compounds 1-A, 1-B, 1-C and 1-D were carried out with HPLC at pH 4 (50 mM sodium acetate buffer) and pH 7.4 (50 mM sodium phosphate buffer). A 730 µM solution of 1-A, 1-B, 1-C or 1-D (concentration stated in doxorubicin equivalents) was incubated at room temperature in the respective buffer system and analyzed by size-exclusion HPLC over 22 h using a Kontron-HPLC system with GeminyxSystem software, column: BioSil SEC250 [300*7,8mm], with a pre-column [80*7,8mm] from Biorad, Germany; flow: 1.2 mL/min, isocratic; injection: 50 µL; mobile phase: 10% acetonitrile / 90% 10 mM sodium phosphate buffer, 0.15 NaCl, pH 7.0, detection at 220 nm and 495 nm.

At pH 4.0 all conjugates 1-A, 1-B, 1-C and 1-D were cleaved and released doxorubicin with the following half-lives:

**Table 2: Half-lives of compounds 1-A, 1-B, 1-C and 1-D at pH 4.0**

| **Conjugate** | **t ½ [h]** |
|---|---|
| Compound 1-A | 3.3 |
| Compound 1-B | 2.1 |
| Compound 1-C | 1.9 |
| Compound 1-D | 2.7 |

In contrast, at pH 7.4 all conjugates were stable with less than 10 % release of doxorubicin over 22 h.

Example 3: *In vivo* efficacy studies were carried out with compounds 1-A, 1-B, and 1-C and doxorubicin in the ovarian carcinoma A2780 xenograft model. For the *in vivo* testing, female NMRI: nu/nu mice (Taconic, Denmark) were used. The mice were held in individually ventilaged cages (IVC) under sterile and standardized environmental conditions (25 ± 2 °C room temperature, 50 ± 10 % relative humidity, 12 hour light-dark-rhythm). They received autoclaved food and bedding (ssniff, Soest, Germany) and acidified (pH 4.0) drinking water *ad libitum.* A2780 tumor fragments were transplanted subcutaneously (s.c.) into the left flank region of anaesthetized (40 mg/kg i.p. Radenarkon, Asta Medica, Frankfurt, Germany) mice on day zero. Mice were randomly distributed to the experimental groups (8 mice per group). When the tumors were grown to a palpable size, treatment was initiated. Mice were treated intravenously with either glucose phosphate buffer (10 mM sodium phosphate, 5 % D-(+)-glucose, pH 5.8), doxorubicin (2 x 8 mg/kg), or compounds 1-A, 1-B, and 1-C (3 x 24 mg/kg doxorubicin equivalents, compounds 1-A, 1-B, and 1-C were dissolved in 10 mM sodium phosphate, 5 % D-(+)-glucose, pH 5.8) at weekly intervals. The injection volume was 0.2 mL/20 g body weight.

Tumor size was measured twice weekly with a caliper-like instrument in two dimensions. Individual tumor volumes (*V*) were calculated by the formula *V* = (length x [width]²)/2 and related to the values on the first day of treatment (relative tumor volume, RTV). Statistical analysis was performed with the U-test (Mann and Whitney) with *p* < 0.05. The body weight of mice was determined every 3 to 4 days.

The results of the experiments in the xenograft tumor models are shown in Fig. 1 and Table 3. The standard and maximum tolerated dose of doxorubicin (2 x 8 mg/kg) was compared to compounds 1-A, 1-B, and 1-C at 3 x 24 mg/kg (doxorubicin equivalents). We and others have shown that 2 x 8 mg/kg doxorubicin is the MTD in nude mice models and higher doses, e.g. 2 x 12 mg/kg, leads to unacceptable toxicity and mortality Kratz et al.: In vitro and in vivo efficacy of acid-sensitive transferrin and albumin doxorubicin conjugates in a human xenograft panel and in the MDA-MB-435 mamma carcinoma model. J. Drug Targeting, 8: 305-318, 2000; Trail et al.: Antigen-specific activity of carcinoma-reactive BR64-doxorubicin conjugates evaluated in vitro and in human tumor xenograft models. Cancer Res., 52: 5693-5700, 1992). In contrast, the compounds 1-A, 1-B, and 1-C could be administered at 3 x 24 mg/kg doxorubicin equivalents without producing mortality or severe body weight loss (see Table 3).

**Table 3: Dose schedule, mortality, body weight change, and antitumor activity of doxorubicin and compounds 1-A, 1-B, and 1-C against human ovarian cancer xenografts (A2780)**

| **Mice** | **Substance** | **Schedule** | **Dose** | **toxic death** | **BWC** | **optimum T/C RTV** |
|---|---|---|---|---|---|---|
| | | [days] | [mg/kg/i.v.] | | [%] | [%] |
| 8 | Glucose-phosphate buffer | 6, 13, 20 | | 0 | -2 | |
| 8 | Doxorubicin | 6, 13 | 8 | 0 | -21 (d26) | 15* |
| 8 | Compound 1-A | 6, 13, 20 | 24 | 0 | -15 (d16) | 0* |
| 8 | Compound1-B | 6, 13, 20 | 24 | 0 | -12 (d13) | 1* |
| 8 | Compound 1-C | 6, 13, 20 | 24 | 0 | -6 (d13) | 1* |

| | | | | | | |
|---|---|---|---|---|---|---|
| * significant versus control group - p < 0.05; BWC = body weight change; T/C RTV = relative tumor volume expressed in % treated versus control groups | | | | | | |

With respect to antitumor efficacy, doxorubicin only showed moderate antitumor efficacy. In contrast, treatment with compounds 1-A, 1-B, and 1-C produced excellent antitumor effects with complete tumor remissions up to day 30 (see Fig. 1) and less body weight change (BWC) than for the doxorubicin treated animals (see Table 3).

## Claims

1. A drug polymer conjugate, comprising a dendritic polyglycerol core with a polyethylene glycol shell, and further comprising at least one pharmaceutically and/or diagnostically active compound, wherein the pharmaceutically and/or diagnostically active compound is bound to said dendritic polyglycerol core through a polymer-binding spacer comprising a polymer-binding moiety and a cleavable linker, and wherein the pharmaceutically and/or diagnostically active compound is not bound to the polyethylene glycol shell.

2. The drug polymer conjugate according to claim 1, wherein the at least one pharmaceutically and/or diagnostically active compound is selected from the group consisting of a cytostatic agent, a cytokine, an immunosuppressant, an antirheumatic, an antiphlogistic, an antibiotic, an analgesic, a virostatic, and an antimycotic agent, a transcription factor inhibitor, a cell cycle modulator, an MDR modulator, a proteasome or protease inhibitor, an apoptosis modulator, an enzyme inhibitor, an angiogenesis inhibitor, a hormone or hormone derivative, a radioactive substance, a light emitting substance, and a light absorbing substance.

3. The drug polymer conjugate according to claim 1 or 2, wherein the at least one pharmaceutically and/or diagnostically active compound is a cytostatic agent selected from the group consisting of N-nitrosoureas; the anthracyclines doxorubicin, daunorubicin, epirubicin, idarubicin, mitoxantrone and ametantrone, 2-pyrollinoanthracyclines, morpholinoanthracyclines, diacetatoxyalkylanthracyclines, and any derivatives thereof; the alkylating agents chlorambucil, bendamustine, melphalan, and oxazaphosphorines, and any derivatives thereof; the antimetabolites 5-fluorouracil, 2'-deoxy-5-fluorouridine, cytarabine, cladribine, fludarabine, pentostatine, gemcitabine and thioguanine, and any derivatives thereof; the folic acid antagonists methotrexate, raltitrexed, pemetrexed and plevitrexed, the taxanes paclitaxel and docetaxel, and any derivatives thereof; the camptothecins topotecan, irinotecan, 9-aminocamptothecin and camptothecin, and any derivatives thereof; the *Vinca* alkaloids vinblastine, vincristine, vindesine and vinorelbine, and any derivatives thereof; calicheamicins and any derivatives thereof; maytansinoids and any derivatives thereof; auristatins and any derivatives thereof; bleomycin, dactinomycin, plicamycin, mitomycin C and cis-configured platinum(II) complexes.

4. The drug polymer conjugate according to any one of claims 1 to 3, wherein the pharmaceutically and/or diagnostically active compound comprises one or more radionuclide(s), one or more positron emitter(s), one or more NMR contrast agent(s), one or more fluorescent compound(s), or one or more near infrared contrast agent(s).

5. The drug polymer conjugate according to any one of claims 1 to 4, wherein said cleavable linker can be cleaved hydrolytically and/or enzymatically and/or pH-dependently.

6. A pharmaceutical composition comprising the drug polymer conjugate according to any one of claims 1 to 5, and optionally a pharmaceutically acceptable carrier and/or a pharmaceutically acceptable adjuvant and/or diluent.

7. The pharmaceutical composition according to claim 6 for the treatment or prevention of a disorder selected from the group consisting of cancer, autoimmune diseases, acute or chronic inflammatory diseases and diseases caused by viruses and/or microorganisms.

8. A kit comprising the drug polymer conjugate according to any one of claims 1 to 5 or the pharmaceutical composition according to claim 6 or 7 and optionally one or more pharmaceutically acceptable adjuvants and/or diluents.

9. The drug polymer conjugate according to any one of claims 1 to 5 for use in treating a patient suffering from a disorder selected from the group consisting of cancer, autoimmune diseases, acute or chronic inflammatory diseases or diseases caused by viruses and/or microorganisms.

## Patentansprüche

1. Arzneimittel-Polymer-Konjugat, umfassend einen dendritischen Polyglycerinkern mit einer Polyethylenglykolhülle und ferner umfassend mindestens eine pharmazeutisch und/oder diagnostisch aktive Verbindung, wobei die pharmazeutisch und/oder diagnostisch aktive Verbindung an den dendritischen Polyglycerinkern über einen polymerbindenden Spacer gebunden ist, der eine polymerbindende Einheit und einen spaltbaren Linker umfasst, und wobei die pharmazeutisch und/oder diagnostisch aktive Verbindung nicht an die Polyethylenglykolhülle gebunden ist.

2. Arzneimittel-Polymer-Konjugat nach Anspruch 1, wobei die mindestens eine pharmazeutisch und/oder diagnostisch aktive Verbindung ausgewählt ist aus der Gruppe, bestehend aus einem Zytostatikum, einem Zytokin, einem Immunsuppressivum, einem Antirheumatikum, einem Antiphlogistikum, einem Antibiotikum, einem Analgetikum, einem Virostatikum und einem Antimykotikum, einem Transkriptionsfaktorinhibitor, einem Zellzyklusmodulator, einem MDR-Modulator, einem Proteasom oder Proteaseinhibitor, einem Apoptosemodulator, einem Enzyminhibitor, einem Angiogeneseinhibitor, einem Hormon oder Hormonderivat, einer radioaktiven Substanz, einer lichtemittierenden Substanz und einer lichtabsorbierenden Substanz.

3. Arzneimittel-Polymer-Konjugat nach Anspruch 1 oder 2, wobei die mindestens eine pharmazeutisch und/oder diagnostisch aktive Verbindung ein Zytostatikum ist, ausgewählt aus der Gruppe, bestehend aus N-Nitrosoharnstoffen; den Anthracyclinen Doxorubicin, Daunorubicin, Epirubicin, Idarubicin, Mitoxantron und Ametantron, 2-Pyrollinoanthracycline, Morpholinoanthracycline, Diacetatoxyalkylanthracycline und jegliche Derivate davon; den Alkylierungsmitteln Chlorambucil, Bendamustin, Melphalan und Oxazaphosphorine und jegliche Derivate davon; den Antimetaboliten 5-Fluoruracil, 2'-Desoxy-5-fluoruridin, Cytarabin, Cladribin, Fludarabin, Pentostatin, Gemcitabin und Thioguanin und jegliche Derivate davon; den Folsäureantagonisten Methotrexat, Raltitrexed, Pemetrexed und Plevitrexed, den Taxanen Paclitaxel und Docetaxel und jegliche Derivate davon; den Camptothecinen Topotecan, Irinotecan, 9-Aminocamptothecin und Camptothecin und jegliche Derivate davon; den Vinca-Alkaloiden Vinblastin, Vincristin, Vindesin und Vinorelbin und jegliche Derivate davon; Calicheamicinen und jegliche Derivate davon; Maytansinoiden und jegliche Derivate davon; Auristatinen und jegliche Derivate davon; Bleomycin, Dactinomycin, Plicamycin, Mitomycin C und cis-konfigurierten Platin(II)-Komplexen.

4. Arzneimittel-Polymer-Konjugat nach einem der Ansprüche 1 bis 3, wobei die pharmazeutisch und/oder diagnostisch aktive Verbindung ein oder mehrere Radionuklid(e), einen oder mehrere Positronenemitter, ein oder mehrere NMR-Kontrastmittel, eine oder mehrere fluoreszierende Verbindung(en) oder ein oder mehrere Nahinfrarot-Kontrastmittel umfasst.

5. Arzneimittel-Polymer-Konjugat nach einem der Ansprüche 1 bis 4, wobei der spaltbare Linker hydrolytisch und/oder enzymatisch und/oder pH-abhängig gespalten werden kann.

6. Pharmazeutische Zusammensetzung, umfassend das Arzneimittel-Polymer-Konjugat nach einem der Ansprüche 1 bis 5 und gegebenenfalls einen pharmazeutisch verträglichen Träger und/oder ein pharmazeutisch verträgliches Adjuvans und/oder Verdünnungsmittel.

7. Pharmazeutische Zusammensetzung nach Anspruch 6 zur Behandlung oder Vorbeugung einer Störung, ausgewählt aus der Gruppe, bestehend aus Krebs, Autoimmunerkrankungen, akuten oder chronischen entzündlichen Erkrankungen und durch Viren und/oder Mikroorganismen verursachten Erkrankungen.

8. Kit, umfassend das Arzneimittel-Polymer-Konjugat nach einem der Ansprüche 1 bis 5 oder die pharmazeutische Zusammensetzung nach Anspruch 6 oder 7 und gegebenenfalls ein oder mehrere pharmazeutisch verträgliche Adjuvantien und/oder Verdünnungsmittel.

9. Arzneimittel-Polymer-Konjugat nach einem der Ansprüche 1 bis 5 zur Verwendung bei der Behandlung eines Patienten, der an einer Störung leidet, die aus der Gruppe ausgewählt ist, bestehend aus Krebs, Autoimmunerkrankungen, akuten oder chronischen entzündlichen Erkrankungen oder durch Viren und/oder Mikroorganismen verursachten Erkrankungen.

## Revendications

1. Conjugué polymère de médicament comprenant un noyau de polyglycérol dendritique avec une coque de polyéthylèneglycol, et comprenant en outre au moins un composé actif du point de vue pharmaceutique et/ou diagnostique, dans lequel le composé actif du point de vue pharmaceutique et/ou diagnostique est fixé audit noyau de polyglycérol dendritique par un écarteur de fixation de polymère comprenant un fragment de fixation de polymère et un liant clivable, et dans lequel le composé actif du point de vue pharmaceutique et/ou diagnostique n'est pas fixé à la coque de polyéthylèneglycol.

2. Conjugué polymère de médicament selon la revendication 1, dans lequel l'au moins un composé actif du point de vue pharmaceutique et/ou diagnostique est sélectionné parmi le groupe constitué d'un agent cytostatique, d'une cytokine, d'un immunosuppresseur, d'un agent antirhumatismal, d'un anti-inflammatoire, d'un antibiotique, d'un analgésique, d'un virostatique et d'un agent antimycosique, d'un inhibiteur du facteur de transcription, d'un modulateur de cycle cellulaire, d'un modulateur MDR, d'un protéasome ou inhibiteur de protéase, d'un modulateur d'apoptose, d'un inhibiteur enzymatique, d'un inhibiteur d'angiogenèse, d'une hormone ou d'un dérivé d'hormone, d'une substance radioactive, d'une substance à émission lumineuse et d'une substance à absorption lumineuse.

3. Conjugué polymère de médicament selon la revendication 1 ou 2, dans lequel l'au moins un composé actif du point de vue pharmaceutique et/ou diagnostique est un agent cytostatique sélectionné parmi le groupe constitué des N-nitrosurées ; des anthracyclines doxorubicine, daunorubicine, épirubicine, idarubicine, mitoxantrone et amétantrone, 2-pyrollinoanthracyclines, morpholinoanthracyclines, diacétatoxyalkylanthracyclines et tout dérivé de celles-ci ; des agents alkylants chlorambucil, bendamustine, melphalan et oxazaphosphorines et tout dérivé de ceux-ci ; des antimétabolites 5-fluorouracile, 2'-désoxy-5-fluorouridine, cytarabine, cladribine, fludarabine, pentostatine, gemcitabine et thioguanine et tout dérivé de ceux-ci ; des antagonistes d'acide folique méthotrexate, raltitrexed, pémétrexed et plevitrexed, des taxanes paclitaxel et docétaxel et tout dérivé de ceux-ci ; des camptothécines topotécane, irinotécane, 9-aminocamptothécine et camptothécine et tout dérivé de celles-ci ; des vinca-alcaloïdes vinblastine, vincristine, vindésine et vinorelbine et tout dérivé de ceux-ci ; des calichéamicines et tout dérivé de celles-ci ; des maytansinoïdes et tout dérivé de ceux-ci ; des auristatines et tout dérivé de celles-ci ; bléomycine, dactinomycine, plicamycine, mitomycine C et complexes de platine(II) à configuration cis.

4. Conjugué polymère de médicament selon l'une quelconque des revendications 1 à 3, dans lequel le composé actif du point de vue pharmaceutique et/ou diagnostique comprend un ou plusieurs radionucléide(s), un ou plusieurs émetteur(s) de positrons, un ou plusieurs agent(s) de contraste RMN, un ou plusieurs composé(s) fluorescent(s) ou un ou plusieurs agent(s) de contraste du proche infrarouge.

5. Conjugué polymère de médicament selon l'une quelconque des revendications 1 à 4, dans lequel ledit liant clivable peut être clivé par voie hydrolytique et/ou enzymatique et/ou dépendante du pH.

6. Composition pharmaceutique comprenant le conjugué polymère de médicament selon l'une quelconque des revendications 1 à 5 et en option un vecteur pharmaceutiquement acceptable et/ou un adjuvant et/ou diluant pharmaceutiquement acceptable.

7. Composition pharmaceutique selon la revendication 6 pour le traitement ou la prévention d'un trouble sélectionné parmi le groupe constitué du cancer, de maladies auto-immunes, de maladies inflammatoires aigues ou chroniques et de maladies provoquées par des virus et/ou micro-organismes.

8. Kit comprenant le conjugué polymère de médicament selon l'une quelconque des revendications 1 à 5 ou la composition pharmaceutique selon la revendication 6 ou 7 et en option un ou plusieurs adjuvants et/ou diluants pharmaceutiquement acceptables.

9. Conjugué polymère de médicament selon l'une quelconque des revendications 1 à 5 destiné à être utilisé dans le traitement d'un patient souffrant d'un trouble sélectionné parmi le groupe constitué du cancer, de maladies auto-immunes, de maladies inflammatoires aigues ou chroniques ou de maladies provoquées par des virus et/ou micro-organismes.
